# EUROPEAN PATENT SPECIFICATION

(11) **EP 0 374 328 B1**
(45) Date of publication and mention of the grant of the patent: **23.03.1994**
(21) Application number: 88312289.7
(22) Date of filing: 23.12.1988
(51) Int. Cl.: H05G 1/60, H04N 5/32, G06F 15/68, A61B 6/00

(54) **X-ray photographic equipment**
Röntgenaufnahmeeinrichtung
Equipement pour la photographie par rayons x

(43) Date of publication of application: 27.06.1990
(73) Proprietor: SHIMADZU CORPORATION, Nakagyo-ku Kyoto-shi Kyoto 604 (JP)
(72) Inventor: Kawai, Masumi, Shimokyoku Kyoto (JP); Sawada, Hiroshi, Ukyoku Kyoto (JP)
(74) Representative: Tribe, Thomas Geoffrey

(56) References cited:
- EP-A- 0 066 824
- EP-A- 0 082 771
- EP-A- 0 102 592
- EP-A- 0 125 130
- EP-A- 0 146 991
- US-A- 4 323 973
- US-A- 4 636 850

## Description

### BACKGROUND OF THE INVENTION

The present invention relates to an X-ray photographic equipment in which an X-ray television is utilized, and more particularly to an X-ray photographic equipment for carrying out digital subtraction angiography (DSA) in which video signals to be processed are digitalized.

When a subject is to undergo DSA in two or more areas of his body, and when these areas are disposed along the direction of flow of a contrast medium, it is most common to carry out DSA with either of the following two techniques, in either of which the contrast medium has only to be injected once for all so as to avoid a cardiodynamic burden which would be imposed on the heart of the subject if the contrast medium were injected in each area:

The first technique comprises the steps of taking a pre-contrast image in one area after another, injecting a contrast medium, taking a contrast image in one area after another, and subtracting the pre-contrast image from the contrast image for each area. A difference obtained from this subtraction represents an image of the contrast medium alone, i.e. an image of the blood vessel alone, the background of which has vanished. Twice in all, an examination table, on which the subject is lying, is moved relative to an image pickup system including an X-ray tube, i.e. once before the injection of the contrast medium and once after the injection thereof.

The second technique begins with taking a pre-contrast image in an area disposed at the upstream side of the flow of a contrast medium. The pre-contrast image is taken before the contrast medium reaches the area. Subsequently, when the contrast medium reaches the area, a contrast image is taken in that area. Then an area disposed at the downstream side is allowed to come into view of the image pickup system, and a pre-contrast image is taken in that area before the contrast medium reaches the area. Subsequently, when the contrast medium reaches the area, a contrast image is taken in that area. Thus the movement of the subject relative to the image pickup system precedes the flow of the contrast medium. In each of a plurality of areas, both a pre-contrast image and a contrast image are taken, and the former is subtracted from the latter.

The first technique is characterized in that, after the first movement of the examination table for the purpose of taking pre-contrast images in a plurality of areas, the examination table is returned to its starting position. The trouble is that, at the time of the second movement, it is difficult to stop the examination table exactly in the same positions as those in which the examination table was stopped at the time of the first movement. Consequently an artefact caused by positional deviation is liable to appear. Even if the examination table per se is free from positional deviation, the body of the subject is liable to deviate from its initial posture in the course of the long interval between the time when a pre-contrast image is taken in an area and the time when a contrast image is taken in the same area. Consequently an artefact caused by postural deviation is also liable to appear.

In the second technique, the examination table has only to be moved once from the uppermost to the downmost side of the contrast medium stream. There is no possibility, therefore, that an artefact caused by positional deviation will appear. It is difficult, however, to start moving the examination table at an opportune moment. The contrast medium may have already reached a second area disposed at the downstream side if the second area is late in coming into view of the image pickup system. In that event, the pre-contrast image will include an image of the contrast medium (i.e. an image of the blood vessel), which will be lost when this pre-contrast image is subtracted from a contrast image. Consequently the portion where the blood vessel exists turns white on an image obtained from such subtraction. As a matter of course, one may contend that the problem of this kind does not arise if the movement of the examination table is started at an opportune moment so that the second area may come into view of the image pickup system before the contrast medium reaches the area. However, this contention does not fit in with the reality, because there is great individual variation as to the velocity of flow of the contrast medium (i.e. the velocity of blood flow).

An X-ray photographic equipment according to the prior art portion of claim 1 is known from EP-A-0 146 991.

### SUMMARY OF THE INVENTION

It is an object of the present invention to provide an X-ray photographic equipment for allowing a subject to undergo DSA in a plurality of areas of his body, these areas being disposed along the direction of flow of a contrast medium, the equipment being capable of delivering such an excellent image as to be free from an artefact caused by positional deviation or from the deficiency of image information.

According to the invention there is provided X-ray photographic equipment comprising:
X-ray radiation means, X-ray image pickup means spaced from said X-ray radiation means so that a body of a subject may be disposed therebetween, said X-ray image pickup means facing said X-ray radiation means so that X-rays produced by said X-ray radiation means may he received by said X-ray image pickup means after passing through the body of the subject, means for moving said X-ray radiation means and said X-ray image pickup means relative to the body of the subject along a direction of flow of a contrast medium in the body of the subject, said moving means including means for stopping said X-ray radiation means and said X-ray image pickup means at a plurality of positions,
and digitizing means for converting each of said image signals into a frame of digital signals representing, pixel-by-pixel, the level of each pixel ranging from black to white, characterised by:
first means responsive to said digitizing means, for producing signals representing a maximum value holding image wherein each pixel has a level equal to the level of the whitest of the corresponding pixels in each of said frames;
second means responsive to said digitizing means, for producing signals representing a minimum value holding image wherein each pixel has a level equal to the level of the blackest of the corresponding pixels in each of said frames, and
difference means responsive to said first means and said second means for producing difference signals representing the difference between the minimum value holding image and the maximum value holding image.

The visual field of the X-ray image pickup means is moved relative to the body of the subject along the direction of flow of the contrast medium in the body of the subject, and stopped in a plurality of positions. During each time when the X-ray image pickup means is at a standstill in an area of the body of the subject, a plurality of images are obtained during the interval between the time when the contrast medium has not reached the area yet and the time when the contrast medium passes through the area. Video signals representing a plurality of images obtained in an area are processed in such a manner that the signal level of each picture element of an image is compared with the signal levels of the corresponding picture elements of other images obtained in the same area. An image which consists of picture elements having the highest signal levels is selected as a maximum value holding image, while an image which consists of picture elements having the lowest signal levels is selected as a minimum value holding image.

The maximum value holding image is represented by the most whitish picture elements. Consequently, even if the signal level undergoes variation in the course of the above-described comparison between the images, an image of the contrast medium moving in the body of the subject does not appear in the maximum value holding image.

On the other hand, the minimum value holding image is represented by the most blackish picture elements. Consequently, even if the signal level undergoes variation in the course of the above-described comparison between the images, a distinct image of the contrast medium moving in the body of the subject appears in the minimum value holding image.

The signal level of an image of the background does not undergo variation in the course of the above-described comparison between the images. Consequently the image of the background appearing in the maximum value holding image is on a level with that appearing in the minimum value holding image.

Consequently a difference between the maximum value holding image and the minimum value holding image represents an image of the contrast medium alone, i.e. an image of the blood vessel alone.

There is no possibility that an artefact caused by positional deviation will appear, because a plurality of images are obtained during the time when the visual field of the X-ray image pickup means is at a standstill relative to the body of the subject.

There is no problem even if the visual field of the X-ray image pickup means is not moved at an opportune moment, i.e., even if the starting of the visual field is delayed. In this case, when the visual field reaches the next area, the contrast medium may have already reached the upstream side of that area. However, the upstream side of an area is a portion from which the contrast medium flows away earliest. Therefore, an image of the contrast medium does not appear in the maximum value holding image at all. Even if the visual field of the X-ray image pickup means is not moved at an opportune moment, there is no possibility that an image of the contrast medium (i.e. an image of the blood vessel) will be lost.

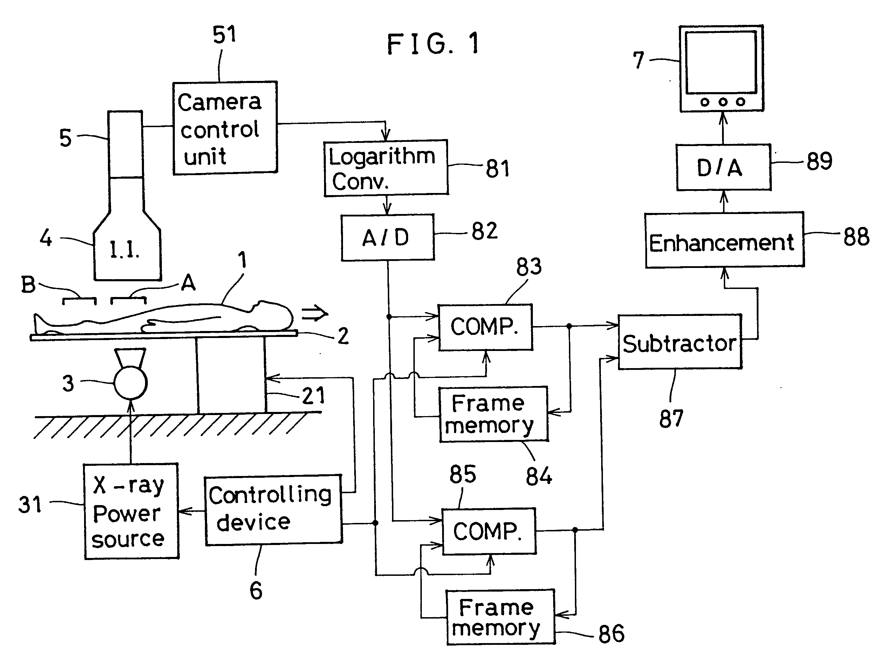

A preferred embodiment of the present invention is hereinafter described with reference to the accompanying drawing.

### BRIEF DESCRIPTION OF THE DRAWING

The single figure of the drawing is a block diagram of an embodiment of the present invention.

### DETAILED DESCRIPTION OF THE INVENTION

Referring to the drawing, a subject 1 is lying on an examination table 2, which is adapted to be moved in the lengthwise direction by a table reciprocating device 21 so as to allow the subject 1 to undergo DSA in two areas A and B of his body. When viewed from the standpoint of the direction in which a contrast medium flows, the area A is disposed at the upstream side and the area B is disposed at the downstream side.

The body of the subject 1 is interposed between an X-ray tube 3 and an image intensifier tube 4. The X-ray tube 3 is supplied with high voltage from a power source 31. An X-ray radiographic image is intensified and converted into a visible image by the image intensifier tube 4 and further converted into a video signal for television by transmission through an image pickup tube 5 and a camera control unit 51.

The table reciprocating device 21 and the power source 31 are controlled by a controlling device 6. The area A is exposed to the irradiation by X rays from the X-ray tube 3 when the examination table is at a standstill under the condition that the area A falls within the visual field of the X-ray image pick-up means. Then a video signal about the area A is developed by the camera control unit 51. In case of an ordinary X-ray fluoroscopic apparatus, this video signal is directly fed to, and displayed on the screen of, a television monitoring device 7.

The area A begins to be exposed to the irradiation by X rays approximately at the time when a contrast medium injected into the subject 1 reaches the area A. Video signals representing a plurality of images are obtained before the contrast medium flows away from the area A. These video signals are fed to an analog-to-digital converter 82 through a logarithmic converter 81 and digitalized. A frame of the digitalized video signals is divided, e.g., into 512 x 512 picture elements. Fifteen bits are used for representing the gradation of each picture element. Then the digital video signals are fed to comparators 83 and 85. Signals having the same picture elements as these digital video signals are read out from the frame memories 84 and 86 and compared with these digital video signals in the comparators 83 and 85. The process of this comparison is started by a control signal taken from the controlling device 6 when the area A begins to be exposed to the irradiation by X rays. Prior to the starting of this process, the minimum (the most blackish) value for each picture element has already been stored in the frame memory 84, while the maximum (the most whitish) value for each picture element has already been stored in the frame memory 86. In the comparator 83, the signal level of each picture element of an image is compared with the signal levels of the corresponding picture elements of other images obtained in the same area, and picture elements having the highest signal levels are selected and stored in memory locations allotted to these picture elements in the frame memory 84. Picture elements having the lowest signal levels are selected by the comparator 85 and stored in the frame memory 86. Thus the process of holding a maximum value is carried out by the comparator 83 and the frame memory 84. When a plurality of images have been outputted from the camera control unit 51, a maximum value holding image which consists of picture elements having the highest signal levels is formed in the frame memory 84. Likewise, the process of holding a minimum value is carried out by the comparator 85 and the frame memory 86. When a plurality of images have been outputted, a minimum value holding image is formed in the frame memory 86.

The maximum value holding image and the minimum value holding image are read out from the frame memories 84 and 86 respectively and subjected to subtraction in a subtractor 87. A difference obtained from this subtraction is fed to a digital-to-analog converter 89 through a contrast enhancement circuit 88, returned to an analog signal, and fed to the television monitoring device 7. The maximum value holding image represents an image of the background alone, in which an image of the contrast medium moving in the body of the subject does not appear at all. On the contrary, the minimum value holding image represents all the loci of the contrast medium moving in the body of the subject. Therefore, the difference obtained from the above-mentioned subtraction represents an image of the contrast medium alone, i.e. an image of the blood vessel alone, which is displayed on the screen of the television monitoring device 7.

Irradiation by X rays is temporarily stopped by the controlling device 6 when an image of the blood vessel has been obtained in the area A. A control signal is fed to the table reciprocating device 21 so as to move the examination table 2 to such an extent that the area B comes to be interposed between the X-ray tube 3 and the image intensifier tube 4. In the area B as in the area A, a maximum value holding image and a minimum value holding image are formed, and a difference therebetween is obtained and displayed. The above-described process of irradiation by X rays, temporary stoppage thereof, and movement of the examination table is subjected to program control by the controlling device 6. Alternatively, only the stoppage of irradiation by X rays and the movement of the examination table may be interlocked, the operator being free to decide on the timing of the stoppage of irradiation.

The processing can proceed in the same manner as mentioned above if there are further areas to be subjected to DSA in the succeeding positions.

Conditions of X-ray photographing are determined by carrying out test irradiation at the outset. It is necessary to determine such conditions for each area, because the thigh A and the shank B are different in thickness from each other. These conditions are stored in the controlling device 6.

The opening or aperture of an automatic diaphragm (not shown) mounted on the incidence side of the image pickup tube 5 is automatically changed so as to allow the quantity of rays incident on the image pickup tube 5 to be kept constant. For this purpose, the opening or aperture of the automatic diaphragm is predetermined on the basis of starting conditions of X-ray photographing obtained from test irradiation. The predetermined opening or aperture of the automatic diaphragm is stored in a memory and automatically changed every time a new area comes to be interposed between the X-ray tube 3 and the image intensifier tube 4.

Although a leg is subjected to DSA in the above-described embodiment, an arm, head or neck may also be subjected to DSA in the same manner as mentioned above.

From the foregoing, it will be apparent that the contrast medium has only to be injected once for all into the body of a subject who is going to undergo DSA in a plurality of areas of his body by means of the X-ray photographic equipment in accordance with the present invention. This equipment is capable of delivering such an excellent image as to be free from the deficiency of indispensable image information or from an artefact caused by positional deviation of the examination table or postural deviation of the body of the subject. The timing for moving the examination table need not be so strict as to require the quick movement thereof or the delicacy in seizing an opportune moment.

## Claims

1. X-ray photographic equipment comprising:
X-ray radiation means (31), X-ray image pickup means (4) spaced from said X-ray radiation means so that a body of a subject (1) may be disposed therebetween, said X-ray image pickup means facing said X-ray radiation means so that X-rays produced by said X-ray radiation means may be received by said X-ray image pickup means after passing through the body of the subject, means (21) for moving said X-ray radiation means and said X-ray image pickup means relative to the body of the subject along a direction of flow of a contrast medium in the body of the subject, said moving means including means (6) for stopping said X-ray radiation means and said X-ray image pickup means at a plurality of positions,
and digitizing means (82) for converting each of said image signals into a frame of digital signals representing, pixel-by-pixel, the level of each pixel ranging from black to white, characterised by:
first means (83,84) responsive to said digitizing means, for producing signals representing a maximum value holding image wherein each pixel has a level equal to the level of the whitest of the corresponding pixels in each of said frames;
second means (85,86) responsive to said digitizing means, for producing signals representing a minimum value holding image wherein each pixel has a level equal to the level of the blackest of the corresponding pixels in each of said frames, and
difference means (87) responsive to said first means and said second means for producing difference signals representing the difference between the minimum value holding image and the maximum value holding image.

2. Equipment as claimed in claim 1, wherein said first means comprises a first frame memory (84) and a first comparator (83), said first comparator having inputs connected to said first frame memory and said digitizing means (82) and an output connected to said first frame memory.

3. Equipment as claimed in claim 2, wherein said first frame memory (84) initially stores signals representing a reference frame wherein each pixel of the reference frame has said black level.

4. Equipment as claimed in claim 3, wherein said second means comprises a second frame memory (86) and a second comparator (85), said second comparator having inputs connected to said second frame memory and said digitizing means (82) and an output connected to said second frame memory.

5. Equipment as claimed in claim 4 wherein said second frame memory initially stores signals representing a second reference frame wherein each pixel of the second reference frame has said white level.

6. Equipment as claimed in claim 1 and further comprising visual display means (7) responsive to said difference means (87) for visually displaying an image representing the difference between the maximum value holding image and the minimum value holding image.

7. Equipment as claimed in claim 6 wherein said visual display means includes a digitial to analog converter (89) connected to said difference means, and a video display (7) connected to said analog to digital converter.

8. Equipment as claimed in claim 1, comprising a logarithm converter (81) disposed before said digitizing means (82).

## Patentansprüche

1. Röntgenaufnahmeeinrichtung umfassend: eine Röntgenstrahlquelle (31), eine von der Röntgenstrahlquelle so beabstandete Röntgenbildaufnahmeeinrichtung (4), daß der Körper einer Person (1) dazwischen angeordnet werden kann, wobei die Röntgenbildaufnahmeeinrichtung der Röntgenstrahlquelle gegenübersteht, so daß die von der Röntgenstrahlquelle erzeugten Röntgenstrahlen von der Röntgenbildaufnahmeeinrichtung aufgenommen werden können, nachdem sie den Körper der Person passiert haben, eine Einrichtung (21) zur Bewegung der Röntgenstrahlquelle und der Röntgenbildaufnahmeeinrichtung relativ zum Körper der Person längs einer Flußrichtung eines Kontrastmittels im Körper der Person, wobei die Bewegungseinrichtung Halteeinrichtungen (6) umfassen, um die Röntgenstrahlquelle und die Röntgenbildaufnahmeeinrichtung in einer Mehrzahl von Positionen anzuhalten und eine Digitalisiereinrichtung (82) zur Konvertierung jedes Bildsignals in ein Raster digitaler Signale, die Bildelement für Bildelement den Pegel jedes Bildelements von schwarz bis weiß wiedergeben, gekennzeichnet durch:
erste Einrichtungen (83, 84), die auf die Digitalisiereinrichtung ansprechen, zur Erzeugung von Signalen, die ein Maximalwerthaltebild erzeugen bei dem jedes Bildelement einen Pegel aufweist der den Pegel des weißesten der entsprechenden Bildelemente jedes Rasters entspricht,
zweite auf die Digitalisiereinrichtung ansprechende Einrichtungen (85, 86) zur Erzeugung von Signalen, die ein Minimalwerthaltebild wiedergeben, in welchem jedes Bildelement einen Pegel aufweist, der dem Pegel des schwärzesten der entsprechenden Bildelemente in jedem dieser Raster entspricht und
eine an die genannten ersten und zweiten Einrichtungen angeschlossene Differenzeinrichtung (87) zur Erzeugung von Differenzsignalen, welche die Differenz zwischen dem Maximalwerthaltebild und dem Minimalwerthaltebild repräsentieren.

2. Einrichtung nach Anspruch 1, dadurch gekennzeichnet, daß die ersten Einrichtungen einen ersten Rasterspeicher (84) und eine erste Vergleichseinrichtung (83) umfassen, wobei die erste Vergleichseinrichtung Eingänge aufweist, die mit dem ersten Rasterspeicher und der Digitalisiereinrichtung (82) verbunden sind und einen Ausgang, der mit dem ersten Rasterspeicher verbunden ist.

3. Einrichtung nach Anspruch 2, dadurch gekennzeichnet, daß der erste Rasterspeicher anfänglich Signale speichert, die ein Referenzraster wiedergeben, in welchem jedes Bildelement des Referenzrasters seinen Schwarzpegel hat.

4. Einrichtung nach Anspruch 3, dadurch gekennzeichnet, daß die zweiten Einrichtungen einen zweiten Rasterspeicher (86) und eine zweite Vergleichseinheit (85) umfassen, wobei die zweite Vergleichseinheit Eingänge aufweist, die mit dem zweiten Rasterspeicher und der Digitalisiereinheit (82) verbunden sind und einen Ausgang, der mit dem zweiten Rasterrahmen verbunden ist.

5. Einrichtung nach Anspruch 4, dadurch gekennzeichnet, daß der zweite Rasterspeicher anfänglich Signale speichert, die ein zweites Referenzraster wiedergeben, in welchem jedes Bildelement des zweiten Referenzrasters den gennanten Weißpegel aufweist.

6. Einrichtung nach Anspruch 1, gekennzeichnet durch eine optische Wiedergabeeinrichtung (7) die auf die Differenzeinrichtung (87) anspricht, um optisch ein Bild anzuzeigen, welches der Differenz zwischen dem Maximalwerthaltebild und dem Minimalwerthaltebild entspricht.

7. Einrichtung nach Anspruch 6, dadurch gekennzeichnet, daß die optische Anzeigeeinrichtung einen Digital-Analogkonverter (89) enthält, der an die Differenzeinrichtung angeschlossen ist und ein Videodisplay (7), welches an den Analog-Digitalkonverter angeschlossen ist.

8. Einrichtung nach Anspruch 1, gekennzeichnet durch einen logarithmischen Konverter (81) der vor der Digitalisiereinrichtung (82) angeordnet ist.

## Revendications

1. Equipement pour la photographie par rayons X comprenant :
des moyens de production de rayons X (31), des moyens de prises d'image par rayons X (4) écartés desdits moyens de production de rayons X de manière que le corps d'un patient (1) puisse être intercalé entre eux, lesdits moyens de prise d'image étant orientés en direction desdits moyens de production de rayons X, de manière que les rayons X produits par lesdits moyens de production soient reçus par lesdits moyens de prise d'image après avoir traversé le corps du patient, des moyens (21) pour déplacer lesdits moyens de production de rayons X et lesdits moyens de prise d'image par rapport au corps du patient dans la direction de circulation d'un produit de contraste dans le corps du patient, lesdits moyens de déplacement comprenant des moyens (6) pour arrêter lesdits moyens de production de rayons X et lesdits moyens de prise d'image en une pluralité de positions, et des moyens de numérisation (82) pour convertir chacun des signaux d'image en une trame de signaux numériques représentant, pixel par pixel, le niveau de chaque pixel s'échelonnant du noir au blanc, caractérisé par :
des premiers moyens (83, 84) répondant auxdits moyens de numérisation, pour produire des signaux représentant une image contenant la valeur maximale dans laquelle chaque pixel a un niveau égal au niveau du plus blanc des pixels correspondants de chacune desdites trames ;
des seconds moyens (85, 86) répondant auxdits moyens de numérisation, pour produire des signaux représentant une image contenant une valeur minimale dans laquelle chaque pixel a un niveau égal au niveau du plus noir des pixels correspondants dans chacune desdites images, et
des moyens de comparaison (87) répondant auxdits premiers moyens et auxdits seconds moyens pour former des signaux de comparaison représentant la différence entre l'image contenant la valeur minimale et l'image contenant la valeur maximale.

2. Equipement selon la revendication 1, dans lequel lesdits premiers moyens comprennent une première mémoire de trame (84) et un premier comparateur (83), ledit premier comparateur ayant des entrées connectées à ladite première mémoire de trame et auxdits moyens de numérisation (82) et une sortie connectée à ladite première mémoire de trame.

3. Equipement selon la revendication 2, dans lequel ladite première mémoire de trame (84) mémorise d'abord des signaux représentant une trame de référence dans laquelle chaque pixel de la trame de référence possède ledit niveau noir.

4. Equipement selon la revendication 3, dans lequel lesdits seconds moyens comprennent une seconde mémoire de trame (86) et un second comparateur (85), ledit second comparateur ayant des entrées connectées à ladite seconde mémoire de trame et auxdits moyens de numérisation (82) et une sortie connectée à ladite seconde mémoire de trame.

5. Equipement selon la revendication 4, dans lequel ladite seconde mémoire de trame mémorise à l'origine des signaux représentant une seconde trame de référence dans laquelle chaque pixel de la seconde trame de référence a ledit niveau blanc.

6. Equipement selon la revendication 1 et comprenant en outre des moyens de représentation visuelle (7) répondant auxdits moyens de comparaison (87) pour présenter une image visuelle affichant la différence entre l'image contenant la valeur maximale et l'image contenant la valeur minimale.

7. Equipement selon la revendication 6, dans lequel lesdits moyens de représentation visuelle comprennent un convertisseur numérique-analogique (89) connecté auxdits moyens de comparaison, et un écran vidéo (7) connecté audit convertisseur analogique-numérique.

8. Equipement selon la revendication 1, comprenant un convertisseur logarithmique (81) placé avant lesdits moyens de numérisation.
